# EUROPEAN PATENT SPECIFICATION

(11) **EP 2 793 450 B1**
(45) Date of publication and mention of the grant of the patent: **15.03.2017**
(21) Application number: 13405047.5
(22) Date of filing: 18.04.2013
(51) Int. Cl.: H04M 1/725, G01D 3/036, G01K 7/42

(54) **System and method for determining sensor accuracy of a portable electronic device**
System und Verfahren zur Bestimmung der Sensorgenauigkeit einer tragbaren elektronischen Vorrichtung
Système et procédé permettant de déterminer la précision du capteur d'un dispositif électronique portable

(43) Date of publication of application: 22.10.2014
(73) Proprietor: Sensirion AG, 8712 Stäfa (CH)
(72) Inventor: Niederberger, Dominik, 8055 Zürich (CH); Sacchetti, Andrea, 8051 Zürich (CH); Böni, Dominic, 8157 Dielsdorf (CH)
(74) Representative: Mirza, Akram Karim

(56) References cited:
- DE-A1- 10 150 823
- US-A1- 2008 143 522
- US-A1- 2011 119 018

## Description

### Technical Field

The present invention relates to a system and a method for determining sensor accuracy in a portable electronic device, and to a portable electronic device incorporating such system.

### Background of the Invention

Sensors integrated into a portable electronic device, particularly into devices not specifically designed for the sole purpose of operating the sensor in question, face very specific problems relating to the accuracy of the sensor measurement. Taking for example a mobile communication device primarily designed to enable voice or data communication over public or private wireless networks it clear that such a device can not be optimized for any of the multiple sensors it may house in the same manner as a purpose specific device. Such devices require additional means to ensure an accurate measurement under a broad variety of circumstances, not all of which can be prescribed by the manufacturer or even predicted.

On the other hand it is known to integrate ambient condition sensors, such as temperature or humidity sensors, which provide a measure of the environment surrounding the device, into portable devices such as mobile phones.
US 2011/119018 A1 refers to an arrangement for the estimation of an ambient temperature of an electronic device. The electronic device comprises a power consuming unit, which is subject to self heating when in use. The arrangement comprises at least a first and a second temperature sensor and a processor. The sensors are adapted to produce first and second temperature measurements as functions of time respectively. A temperature transport time to the first sensor from the power consuming unit differs from a temperature transport time to the second sensor from the power consuming unit. The processor is adapted to determine an ambient temperature estimate based at least on the first and second temperature measurements as functions of time.
DE 101 50 823 A1 discloses a mobile telecommunication terminal comprising interfaces which allow information to be communicated to the user. There is a sensor provided to measure weather forecast. An interface contains devices allowing the user to access such information from a mobile phone. There are pressure sensors to deduce weather development. There are means to activate a memorisation of pressure according to which a calculation of an altitude can be carried out. The terminal can transfer the information to a server.

In US 2008/143522 A1, a portable computer and a mobile phone are provided. The portable computer includes a data processing module, an environment parameter sensor and a screen. The environment parameter sensor is electrically connected to the data processing module to transmit a sensed environment parameter to the data processing module. The screen is electrically connected to the data processing module to show the environment parameter. The mobile phone includes a control module, a communication module, an environment parameter sensor and a screen. The communication module is electrically connected to the control module. The environment parameter sensor is electrically connected to the control module to transmit a sensed environment parameter to the control module. The screen is electrically connected to the control module to show the environment parameter.

In the light of the above it is seen as an object of the invention to improve an ambient condition sensor such as temperature sensors in portable electronic device and the methods for using such sensors.

### Summary of the Invention

In accordance with an aspect of the invention there is provided a portable electronic device with one or more integrated ambient condition sensors, such as temperature sensors, for measuring parameters characterizing the surrounding of the portable device such as temperature, a display for displaying temperature related information and shared by other elements of the device, and a system receiving input relating to internal or external states of the device and generating in response to the input an accuracy measure of the temperature measurement.

The ambient condition sensor is preferably a sensor where the sensor components are integrated with analog and digital signal processing circuitry on a shared CMOS-type silicon substrate.

The input relating to internal or external states of the device can for example include signals representative of changes in the magnitude of the ambient parameter measured, such as the temperature as measured by a temperature sensor(s), or signals derived from such changes, signals representative of changes in the load or use of internal components of the device, or changes in the location, movements or orientation of the device and any combination of such signals. The signals representative of changes in the temperature or humidity as measured by the temperature sensor(s) may also include signals as derived from a compensation system designed to reduce the difference between the temperature as measured by the integrated sensor and the actual ambient temperature to be determined by the measurement.

The accuracy measure can be a qualitative or quantitative indicator readily transformable into a form which can be displayed using the shared display. The accuracy measure may be transformed into a signal string matching a classification or index system as provided by the general operating system of the device, or into a message to a user indicating that the measurement by the sensor cannot be performed, a holding message indicating that a more accurate reading is attempted or providing instructions for repeating the measurement.

The portable electronic device can be a mobile phone, a handheld computer, an electronic reader, a tablet computer, a game controller, a pointing device, a photo or a video camera, a digital music player, a wrist watch, a key fob, a head set, a digital photo frame and a computer peripheral.

Other advantageous embodiments are listed in the dependent claims as well as in the description below. The described embodiments similarly pertain to the device, the method, and any computer program elements. Synergetic effects may arise from different combinations of the embodiments although they might not be described in detail.

Further on it shall be noted that all embodiments of the present invention concerning a method might be carried out in the order of the steps as described. Nevertheless this has not to be the only essential order of steps but all different orders of the method steps where technically feasible shall be comprised in the scope of the claims and be disclosed by the method claims.

### Brief Description of the Drawings

The detailed description refers to examples of the present invention making reference to the annexed drawings, wherein:
FIG. 1A is a perspective view of a portable electronic device;
FIG. 1B is a schematic view into part of the housing of the device of Fig. 1A;
FIG. 2 is a block diagram with components of a portable device in accordance with an example of the invention;
FIG. 3A shows a block diagram illustrating an example of the invention; and
FIGs. 3B and 3C show different examples of input parameters for an example of the invention.

### Detailed Description

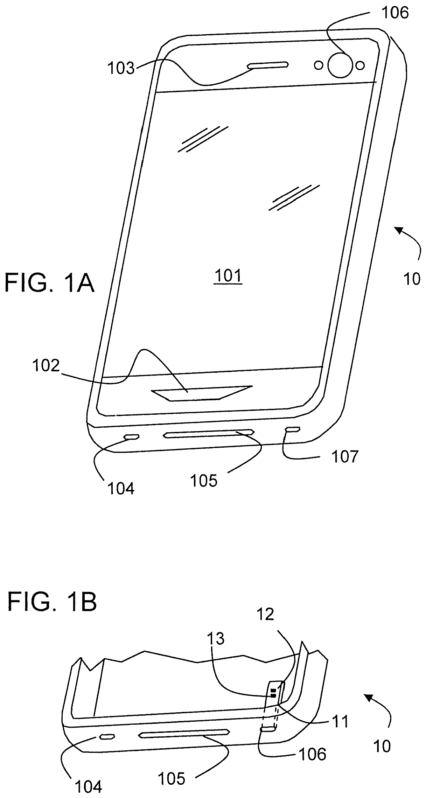

The device of FIG. 1A is a portable electronic device such as a mobile phone. The housing 10 of the mobile phone includes a front side with a screen 101 and elements like buttons 102 to let a user interact with the phone. Also shown on the front side is an opening 103 for a loudspeaker. Further openings 104,105 are located at a lower side wall of the housing 10. It is well known to mount components like microphones and loudspeakers behind such openings. The phone includes one or two cameras 106, and internally additional sensors (not shown) such as location sensors or GPS, and acceleration and orientation sensors in a manner well known.

Another opening 107 is located at the lower side wall. As shown in FIG. 1B the opening 107 is linked to a tubular duct 11 passing through the interior of the housing. A temperature sensor 12 and a humidity sensor 13 are both mounted along the duct 11 such that the sensitive areas of both sensors are exposed to the ambient air through the opening 107. Suitable sensors are commercially available for example from Sensirion™ AG under the tradenames SHTC1 or STS21 (as temperature only sensor). The actual size and shape of the duct 11 depends on the volume available and the nature of the temperature sensor 12 and the humidity sensor 13 can vary, but given the physical constraints of portable mobile devices the area of the opening is typically in the range of less than 10 square millimeters and in the present example actually about less than 3.1 square millimeters.

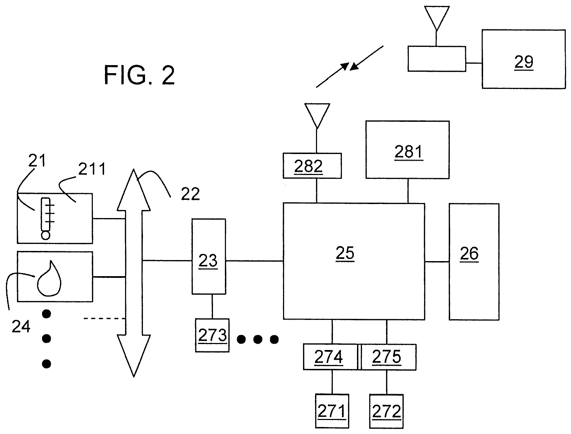

Fig. 2 shows a block diagram with the most important components of the portable device. In particular, the device includes a temperature sensor 21 integrated as part of a CMOS substrate 211 which has CMOS circuitry to control the basic functions and the basic readout of the sensor. The CMOS circuit can include for example the driver to switch the sensor and his heater on or off as well as A/D converters and amplifiers and an I2C bus controller to exchange data on an I2C bus 22. The I2C bus connects the sensors with a sensor hub 23. A further humidity sensor 24 is also linked to the I2C bus 22. The sensor hub 23 provides a control and processing unit for more complex control and read-out functions of the temperature sensor 21 based on signals sent to or extracted from, respectively, the on-chip CMOS circuitry. The sensor hub 23 also controls other auxiliary sensors such as GPS, magnetometers, accelerometers and the like.

Further control and read-out function can also be performed by the central processing unit (CPU) 25 of the portable device, which in turn has read/write access to a memory 26, which can include static or volatile memory or both as known in the art. The memory 26 typically stores the operating system of the device and can also be used to store application programs specific to the operation of the sensors of the portable device. The functions performed by the sensor hub and the sensor specific programs and program libraries as stored and executed by the CPU 25 form a temperature and humidity processing unit capable of transforming the measurements of the sensor into a result which can be displayed or otherwise communicated to the user of the portable device.

It can also be used to store executable code for a compensator used to correct the temperature or humidity as directly measured by the temperature sensor to compensate for effects of the surrounding of the sensor inside the phone or external to it. Such a compensator includes typically a representation of a model which takes into account heat sources, heat capacities and heat conduction of elements inside the device, its housing and other factors. Based on this model and measurements relating to present status of the elements, the measured temperature value is corrected before being displayed.

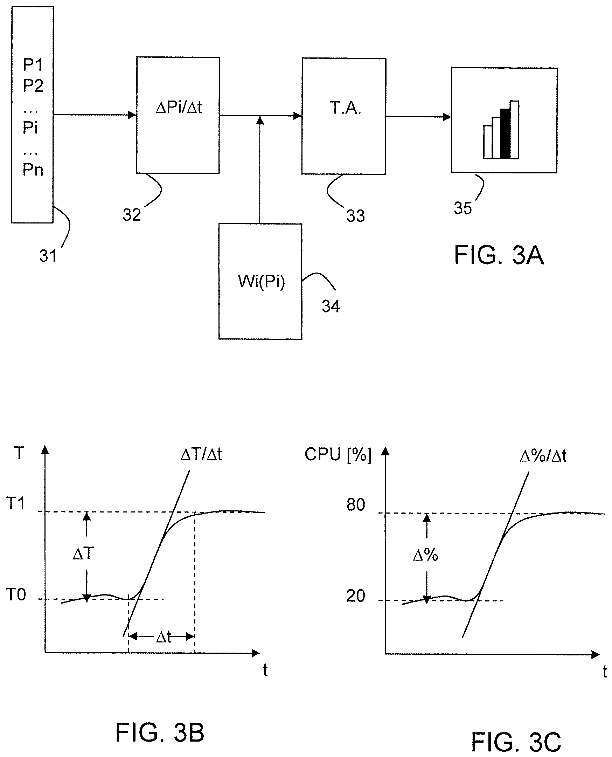

In the present example the CPU 25 and the memory 26 include and execute a sensor accuracy indicator in form of executable code. Functions of the sensor accuracy indicator are described in more detail below while making reference to FIG. 3 using the example of a temature sensor and measurement as ambient condition sensor and ambient parameter.

In addition to the specific sensors as described above, the CPU is also connected to one or more sensors, for example the camera 271 or the microphone 272 also shown as the camera 106 and the microphone 104 of FIG. 1. Other sensors 273 such as location, acceleration and orientation sensors can be controlled by the sensor hub 23 as shown in the example. The sensors 271, 272 communicate with the CPU using their own interface units 274, 275, respectively, which operate typically in complete independence of the temperature sensor 21.

The device includes further well known input/output units 281 such as a touch sensitive display, virtual or physical keyboards and gesture tracking devices etc. The portable device as shown has a telecommunication circuit 282 comprising an antenna, driver circuits and encoding and decoding units as are well known in the art. Using such a telecommunication circuit, the device can connect to a public voice and data network and remote locations 29 as shown.

The diagrams of FIG. 3 illustrate elements of the temperature accuracy indicator. Whilst realized in form of executable code in the present example, the functional elements of the system can be implemented in other known forms of software, firmware or hardware. It should further be noted that some or all of the elements and their respective implementation can be also realized as dedicated microprocessors programmed accordingly.

Generally speaking, in the present example as illustrated in FIG. 3A the temperature accuracy indicator takes as input 31 one or more parameters P1...Pn relevant to the temperature measurement. From these parameters a measure 32 for the change rate ΔPi/Δt or speed of change of one or more these parameters P1...Pn is derived. This change rate is transformed into an accuracy measure 33 or T.A. The transformation can make use of a relevance matrix 34 which allocates a weight Wi to the parameter Pi or to a parameter change. The relevance matrix reflects prior knowledge as to the influence any particular parameter or its change has on the accuracy of the temperature measurement.

The result T.A. of the accuracy measurement 33 can in turn be translated into a form 35 which is easily displayable on the device display.

Typically the calculation is a multi-variant problem where the accuracy is measured as a function of many parameters Pi simultaneously, the time t to determine change rates and the weights Wi which reflect the contribution of each parameter to the accuracy. For display the result of the calculation of the accuracy measure requires typically a further transformation into a normalized number range, e.g., 1 to 10, a bar chart, a color code, e.g., green to red or a combination of such forms suitable for display.

The possible number of parameters influencing the accuracy of the temperature sensor is large and includes parameters internal to the sensor such as its thermal inertia or latency, which can cause inaccuracies during a sudden change in temperature.

Another group of parameters which influence the temperature measurements are internal to the device and its operation, These parameters can for example represent the temperature changes caused by internal heat sources such as CPU, display, radio, camera, speaker, flash lights, GPS, (dis-)charging of the battery etc. Parameters relating to the operation of these internal components can be typically gained from the operating system of the device.

A third group of parameters are parameters external to the device such as representing inaccuracies caused by its handling, its location and exposure to the environment. As far as information relating to such parameters can be gained from the temperature or others sensors and components of the device, they can be parameterized and included into the above process. The parameterization can often include a model to translate readings from the sensors and other components sensor into a parameter relevant for the accuracy of the temperature measurement. As an example a brightness sensor in the phone can be used to detect the presence of direct sunlight on the device causing possibly a deviation of the actual ambient temperature from the temperature as measured.

Examples of such parameters are illustrated in FIGs. 3B and 3C, e.g. a parameter internal to the temperature sensor, itself, namely the temperature measurement itself, and a parameter representing an internal component, namely the CPU load, respectively.

In FIG. 3C the temperature curve 41 representing measurements of the temperature sensor integrated into the device show a change in temperature from a temperature T0 to a temperature T1. The difference between the two temperatures is ΔT. The absolute value of the change can be used in the calculation of the accuracy reflecting the observation that the larger the jump the less accurate the temperature reading is (at least temporarily until the device reaches a steady state again). Neglecting for simplicity any other parameters, the difference ΔT can be multiplied with a weight Wt and transformed into an accuracy measure.

Similarly, the rate ΔT/Δt at which a change takes place can be used as a measure for the accuracy of the temperature measurement. The change rate can be set for example proportional to the accuracy T.A. of the temperature measurement resulting (again in a simplified case) in relations such as T.A. = Wt* ΔT/Δt, where the weight Wt is taken as the proportionality constant. Using several parameters the accuracy T.A. could be for example calculated by a sum of such terms.

Whilst the temperature measurement is directly accessible from the sensor or sensor hub as described above, parameters which are linked to other components of the device are often accessible through calls to routines or stat files of the operating system.

A use of a total CPU load is shown in the example of FIG. 3C. In the example the CPU load in percentage of full load jumps from 20% to 80%. The increase in CPU load generates a heat spike in the phone rendering the temperature measurement less accurate. After testing or modeling by how much such a jump influences the accuracy of the measurement and defining a weight W% based thereon, a similar relationship as in the case of temperature jumps can be defined by T.A. = W%*Δ%/Δt.

The usage or load of other elements such as the radio components or the display of the phone can be used and their contribution to the (lack of) accuracy dealt with in similar manner. It is also possible to use the absolute values of parameters which are linked to other components of the device as input. Hence a load level of 80% of the CPU can be transformed into an accuracy measure and a load of 20% into a different accuracy measure. Typically, it is found that the higher the load on such components the less accurate the result of the ambient sensor measurement. These possible input can be refined using for example the duration of the time at which a load remains at a given level.

After testing or modeling by how much external states such as direct sunlight on the phone can influence the temperature measurement, their contributions to the calculation of accuracy T.A. can be handled in a similar manner. It should however be noted that such modeling of external states of the phone is more complex than the load measurements of internal components and can involve the combination of several sensors or data. For example the influence of the sunlight can depend on the time and the location of the phone to yield a more accurate measure on the sun position and intensity. The external states can also be used for example to detect whether the portable device is kept in a pocket, held in a hand, or placed on a surface. These states can correspond to different accuracy levels of the measurement.

The extent to which the model and hence the accuracy of weighting factors Wi in the above processing steps is a matter of capacity and desired accuracy of the calculation. It should be further noted that the parameters can be instantaneous or averaged parameters to exclude for example variations with frequencies much higher than the relevant time scale of the temperature measurement.

In cases where the device includes means for compensating the temperature measurement to correct for the detrimental influences of the parameters as outlined above, outputs of such a compensator can be used to derive a measure of the accuracy T.A. of the temperature measurement. Using for example the difference between the value of the temperature as measured by the temperature sensor and the value of the temperature as corrected by the compensator the accuracy can be defined as directly proportional to it. Alternatively the time variations or change rate of this difference can be used.

The accuracy measure T.A. as gained by the processes described above or other similar processes can be used in a variety of ways. It is for example possible to transform the measure into a message to the user or a system command to the device itself. The messages include for example the display 35 as number or bar chart, color or other symbols. The messages can use system calls, routines or libraries as typically provided by the operating system of the device or its extensions. In such a case the transformation includes a mapping of T.A. values into a format as required by specific call, routine or library used.

The messages generated based on the value of T.A. can also include instructions to the user as to remove the causes of the inaccuracy such as message to switch off concurrently running processes of the phone or wait for their termination. Other messages could include instructions such as recommending a change of the handling or orientation of the phone or a request for repeating the measurement.

Many of the above examples apply equally or in modified from to other ambient sensor measurements such as humidity. For example the humidity measurement is also known to be less accurate at bigger steps in its absolute value than at smaller steps. As far as the humidity measurement R.H. includes a temperature measurement for the determination of its absolute value, e.g., R.H. = R.H.(T), any inaccuracy of the temperature measurement T.A. relates to an inaccuracy of the humidity in the order which is determined by the functional relationship R.H. (T) .

While there are shown and described presently preferred embodiments of the invention, it is to be understood that the invention is not limited thereto but may be otherwise variously embodied and practised within the scope of the following claims.

## Claims

1. A portable electronic device comprising an integrated temperature sensor (12, 21,) for measuring an ambient temperature, a display (101) for displaying the temperature or related information and shared by other elements of the device, and a temperature sensor accuracy system (25, 26) adapted to receive inputs (31) in form of parameters Pi relevant to the temperature measurement and relating to internal or external states of the device and to generate in response to the inputs (31) an accuracy measure (33) of the temperature measurement, by deriving a measure for a change rate ΔPi/Δt or a speed of change of one or more of these parameters Pi, and by transforming these one or more change rates into the accuracy measure (33) by making use of a relevance matrix (34) which allocates a weight to the parameter or parameter change.

2. The device according to claim 1, wherein the inputs (31) relating to internal or external states of the device include parameters relating to measurements of the temperatures sensor (12, 21), parameters relating the usage or load of other elements of the device, parameters relating to the handling, location, orientation or movement of the device or a combination of such parameters.

3. The device according to claim 1 or 2, further comprising a storage (26) storing a library of values or functions defining the relative contribution of each parameter Pi or parameter change ΔPi/Δt or of a group of parameters Pi or parameter changes ΔPi/Δt to the generation of the accuracy measure (33) with said storage (26) being linkable to the temperature sensor accuracy system (25, 26).

4. The device according to any of the preceding claims, further comprising a converter for transforming the accuracy measure (33) into a message for being displayed on the display (101).

5. The device according to claim 4, wherein the converter maps the accuracy measure (33) into a message format as provided by an operating system of the device.

6. The device according to any of the preceding claims, further comprising a compensator for correcting the ambient sensor measurement with the compensator providing an input to the ambient sensor accuracy system (25, 26).

7. The device according to any of the preceding claims, wherein components of the temperature sensor (12, 21,) are integrated with analog and digital signal processing circuitry on a shared CMOS-type silicon substrate.

8. The portable electronic device according to any of the preceding claims, being selected from a group comprising:
a mobile phone,
a handheld computer,
an electronic reader,
a tablet computer,
a game controller,
a pointing device,
a photo or a video camera,
a digital music player,
a wrist watch,
a key fob,
a head set,
a digital photo frame,
and a computer peripheral.

9. A method for determining the accuracy of an ambient temperature measurement taken by a temperature sensor integrated into a portable electronic device, the method comprising the steps of measuring the ambient temperature using the integrated temperature sensor (12, 21,), providing inputs (31) in form of parameters Pi relevant to the measurement and relating to internal or external states of the device to a temperature sensor accuracy system (25, 26) in the device, and generating in response to the input an accuracy measure (33) of the ambient temperature measurement, wherein the step of generating the accuracy measure (33) includes
- deriving a measure for a change rate ΔPi/Δt or a speed of change of one or more of these parameters Pi,
- transforming the one or more change rates into the accuracy measure (33) by making use of a relevance matrix (34) which allocates a weight to the parameter or parameter change.

10. The method according to claim 9, further comprising the step of transforming the accuracy measure (33) into a message for being displayed on a display (101) of the device.

11. The method according to claim 10, wherein the accuracy measure (33) is mapped into a message format as provided by an operating system of the device.

12. The method according to any of the claims 9 to 11, wherein the inputs (31) relating to internal or external states of the device include inputs (31) provided by a compensator for correcting the ambient temperature sensor measurement.

## Patentansprüche

1. Eine tragbare elektronische Vorrichtung umfassend einen integrierten Temperatursensor (12, 21) zur Messung einer Umgebungstemperatur, eine Anzeige (101) zum Anzeigen der Temperatur oder von verwandten Informationen, welche von anderen Elementen der Vorrichtung geteilt wird, und ein Temperatursensorpräzisionssystem (25, 26), ausgestaltet zum Empfangen von Eingaben (31) in Form von Parametern Pi, die relevant für die Temperaturmessung sind und die auf interne oder externe Zustände der Vorrichtung bezogen sind, und als Antwort auf die Eingaben (31) eine Präzisionsmessung (33) der Temperaturmessung zu generieren, indem es ein Mass für eine Änderungsrate ΔPi/Δt oder eine Geschwindigkeit einer Änderung eines oder mehrerer dieser Parameter Pi herleitet, und indem diese eine oder mehreren Änderungsraten in die Präzisionsmessung (33) umgewandelt werden, indem eine Relevanzmatrix (34) verwendet wird, welche dem Parameter oder der Parameteränderung eine Gewichtung zuordnet.

2. Die Vorrichtung nach Anspruch 1, wobei die Eingaben (31), die auf interne oder externe Zustände der Vorrichtung bezogen sind, Parameter die sich auf Messungen des Temperatursensors (12, 21) beziehen, Parameter die sich auf die Verwendung oder die Last anderer Elemente der Vorrichtung beziehen, Parameter die sich auf die Handhabung, den Standort, die Ausrichtung oder die Bewegung der Vorrichtung beziehen, oder eine Kombination solcher Parameter, einschliessen.

3. Die Vorrichtung nach Anspruch 1 oder 2, weiter umfassend einen Speicher (26) der eine Bibliothek von Werten oder Funktionen speichert, die den relativen Beitrag jedes Parameters Pi oder jeder Parameteränderung ΔPi/Δt oder einer Gruppe von Parametern Pi oder Parameteränderungen ΔPi/Δt zur Generierung der Präzisionsmessung (33) definieren, wobei der Speicher (26) mit dem Temperatursensorpräzisionssystem (25, 26) verknüpfbar ist.

4. Die Vorrichtung nach einem der vorangehenden Ansprüche, weiter umfassend einen Konverter zur Umwandlung der Präzisionsmessung (33) in einer Nachricht die auf der Anzeige (101) angezeigt werden soll.

5. Die Vorrichtung nach Anspruch 4, wobei der Konverter die Präzisionsmessung (33) in ein Nachrichtenformat überträgt, das von einem Betriebssystem der Vorrichtung bereitgestellt ist.

6. Die Vorrichtung nach einem der vorangehenden Ansprüche, weiter umfassend einen Kompensator zum Korrigieren der Umgebungsmessung des Sensors, wobei der Kompensator eine Eingabe zum Temperatursensorpräzisionssystem (25, 26) bereitstellt.

7. Die Vorrichtung nach einem der vorangehenden Ansprüche, wobei Komponenten des Temperatursensors (12, 21) mit analogen und digitalen Signalverarbeitungsschaltungen auf einem geteilten CMOS-Typ Silikonsubstrat integriert sind.

8. Die tragbare elektronische Vorrichtung nach einem der vorangehenden Ansprüche, ausgewählt aus einer Gruppe umfassend:
ein Mobiltelefon,
einen Taschencomputer,
ein elektronisches Lesegerät,
ein Tablett,
eine Spielkonsole,
ein Zeigegerät,
eine Foto- oder eine Videokamera,
ein digitales Abspielgerät,
eine Armbanduhr,
einen Schlüsselanhänger,
ein Headset,
einen digitalen Bilderrahmen,
und eine Computerperipherie.

9. Ein Verfahren zur Bestimmung der Präzision einer Umgebungstemperaturmessung, die von einem Temperatursensor, der in einer tragbaren elektronischen Vorrichtung integriert ist, durchgeführt wird, wobei das Verfahren die Schritte umfasst:
Messung der Umgebungstemperatur unter Verwendung des integrierten Temperatursensors (12, 21),
Bereitstellung von Eingaben (31) in Form von Parametern Pi, die relevant für die Temperaturmessung sind und die auf interne oder externe Zustände der Vorrichtung bezogen sind, für ein Temperatursensorpräzisionssystem (25, 26) in der Vorrichtung, und
Generieren, als Antwort auf die Eingabe, einer Präzisionsmessung (33) der Umgebungstemperaturmessung, wobei der Schritt der Generierung der Präzisionsmessung (33)
- ein Herleiten eines Masses für eine Änderungsrate ΔPi/Δt oder einer Geschwindigkeit einer Änderung eines oder mehrerer dieser Parameter Pi,
- ein Umwandeln der einen oder mehreren Änderungsraten in die Präzisionsmessung (33), indem eine Relevanzmatrix (34) verwendet wird, welche dem Parameter oder der Parameteränderung eine Gewichtung zuordnet, einschliesst.

10. Das Verfahren nach Anspruch 9, weiter umfassend den Schritt des Transformierens der Präzisionsmessung (33) in eine Nachricht die auf der Anzeige (101) angezeigt werden soll.

11. Das Verfahren nach Anspruch 10, wobei die Präzisionsmessung (33) in ein Nachrichtenformat übertragen wird, das von einem Betriebssystem der Vorrichtung bereitgestellt ist.

12. Das Verfahren nach einem der Ansprüche 9 bis 11, wobei die Eingaben (31), die die auf interne oder externe Zustände der Vorrichtung bezogen sind Eingaben (31) umfassen, die von einem Kompensator zum Korrigieren der Umgebungsmessung des Sensors bereitgestellt werden.

## Revendications

1. Un dispositif électronique portable comprenant un capteur de température intégré (12, 21) pour mesurer une température ambiante, un affichage (101) pour afficher la température ou des informations pertinentes et partagées par d'autres éléments du dispositif, et un système de précision du capteur de température (25, 26), adapté à recevoir des entrées (31) en forme de paramètres Pi pertinents pour la mesure de température et liés à des états internes ou externes du dispositif et à générer une mesure de précision (33) de la mesure de température en réponse aux entrées (31), en dérivant une mesure pour un taux de changement ΔPi/Δt ou une vitesse de changement d'un ou de plusieurs de ces paramètres Pi, et en transformant l'un ou les plusieurs taux de changement en une mesure de précision (33) en utilisant une matrice de pertinence (34) qui attribue une pondération au changement du paramètre ou des paramètres.

2. Le dispositif selon la revendication 1, les entrées (31) étant liées à des états internes ou externes du dispositif incluant des paramètres liés à des mesures du capteur de température (12, 21), des paramètres liés à l'utilisation ou charge d'autres éléments du dispositif, des paramètres liés à la manipulation, location, orientation ou mouvement du dispositif ou à une combinaison de tels paramètres.

3. Le dispositif alimentaire selon la revendication 1 ou 2, comprenant en outre une mémoire (26) qui mémorise une bibliothèque de valeurs ou fonctions qui définissent la contribution relative de chaque paramètre Pi ou changement de paramètres ΔPi/Δt ou d'un groupe de paramètres Pi ou de changements de paramètres ΔPi/Δt à la génération de la mesure de précision (33), la mémoire (26) étant liée au système de précision du capteur de température (25, 26).

4. Le dispositif selon l'une des revendications précédentes, comprenant en outre un convertisseur pour transformer la mesure de précision (33) en un message pour être affiché sur l'affichage (101).

5. Le dispositif selon la revendication 4, le convertisseur reproduisant la mesure de précision (33) dans un format de message prévu par le système de fonctionnement du dispositif.

6. Le dispositif selon l'une des revendications précédentes, comprenant en outre un compensateur pour corriger la mesure ambiante du capteur, le compensateur fournissant une entrée au système de précision du capteur de température (25, 26).

7. Le dispositif selon l'une des revendications précédentes, des composantes du capteur de température (12, 21) étant intégrées avec des circuits de traitement de signaux analogues et digitaux sur un substrat de silicone du type CMOS partagé.

8. Le dispositif électronique portable selon l'une des revendications précédentes, étant sélectionné du groupe comprenant:
un téléphone portable,
un ordinateur portable,
un lecteur électronique,
une tablette,
un contrôleur de jeu,
un dispositif de pointage,
une caméra photo ou vidéo,
un lecteur de musique digital,
une montre à bracelet,
un porte-clés,
une casque-micro,
un cadre de photos,
et une périphérie d'ordinateur.

9. Un procédé pour déterminer la précision d'une mesure de température ambiante prise par un capteur de température intégré dans un dispositif électronique portable, le procédé comprenant les étapes de mesurer la température ambiante en utilisant le capteur de température intégré (12, 21), fournissant des entrées (31) en forme de paramètres Pi, pertinents pour la mesure de température et liés à des états internes ou externes du dispositif, à un système de précision du capteur de température (25, 26) dans le dispositif, et de générer une mesure de précision (33) de la mesure de température, les étapes de génération de la mesure de précision (33) incluant
- dériver une mesure pour un taux de changement ΔPi/Δt ou une vitesse de changement d'un ou de plusieurs de ces paramètres Pi,
- de transformer l'un ou les plusieurs taux de changement en une mesure de précision (33) en utilisant une matrice de pertinence (34) qui attribue une pondération au changement du paramètre ou des paramètres.

10. Le procédé alimentaire selon la revendication 9, comprenant l'étape de transformer la mesure de précision (33) en un message pour être affiché sur l'affichage (101) du dispositif.

11. Le procédé alimentaire selon la revendication 10, la mesure de précision (33) étant reproduite dans un format de message prévu par le système de fonctionnement du dispositif.

12. Le procédé selon l'une des revendications 9 à 11, les entrées (31) liées à des états internes ou externes du dispositif incluant des entrées (31) fournies par un compensateur pour corriger la mesure de température ambiante du capteur.
